# EUROPEAN PATENT APPLICATION

(11) **EP 0 537 936 A1**
(43) Date of publication of application: **21.04.1993**
(21) Application number: 92309118.5
(22) Date of filing: 07.10.1992
(51) Int. Cl.: A61B 5/00, A61N 1/38, G06F 15/20

(54) **Facsimile transfer of electrocardiographic data**

(30) Priority: 17.10.1991 US 778405
(71) Applicant: PHYSIO-CONTROL CORPORATION, Redmond Washington 98052-1013 (US)
(72) Inventor: Morgan, Carlton B., Bainbridge Island, Washington 98110 (US)
(74) Representative: Spall, Christopher John

(57) **Abstract**

Disclosed is a medical instrument that operates to record physiological data indicative of a patient's physical condition as well as event data indicative of the functions performed by the instrument. The instrument (10) includes a microprocessor (20), which produces a patient report by inserting the recorded physiological data and event data into a template of the patient report that is stored in a ROM (42). The microprocessor formats the patient report into a series of facsimile compatible codes so that the patient report can be transmitted by a modem and telephone interface (72) to a remote facsimile machine. Also included is a thermal printer (70) that enables the microprocessor to print facsimile reports from signals received from the telephone interface. In the preferred embodiment, the instrument also includes an electrocardiogram monitor (26) that produces ECG signals indicative of the patient's electrocardiological signals, and a defibrillation circuit (45) that delivers a defibrillation pulse to the patient as required.

## Description

### Field of the Invention

The present invention relates to medical instruments in general, and in particular, to a medical instrument that monitors physiological data for a patient and transmits the data to a remote location.

### Background of the Invention

Medical instruments are available to monitor and record the physiological condition of a patient, and typically produce reports comprising strings of digitally sampled physiological data, event codes that represent the functions performed by the instrument, and time stamps indicating the time at which a certain function was performed. Such records can be reconstructed into a human readable form at a later date by printing descriptive phrases that correspond to the event codes along with the time stamps to indicate the time at which such events took place. Also, many such medical instruments are capable of printing a strip chart record of the physiological data taken from the patient. The printed records can then be used to assess the operation of the instrument, and the effectiveness of the medical treatment technique used. The report may also be used to assess the quality of care being delivered by the instrument's operators, and used to provide quality assurance feedback to these care providers. An equally important use for such reports is to transmit data from an emergency medical team to a remotely located physician so that the physician can instruct the team how to treat the patient.

Even if a physician is in radio or telephone contact with the emergency medical team who is treating the patient, there is always a chance of a miscommunication between the physician and the emergency medical team or of a misunderstanding regarding the patient's condition due to varying interpretations of the physiological data by the emergency medical team. Several systems have been developed to decrease the likelihood of error regarding a patient's physical condition. For example, medical instruments have been developed that record a patient's electrocardiogram (ECG) signal and transmit it to a remote receiver that prints a strip chart of the ECG signal for the physician to review. The transmission may use either a radio signal or a telephone line to convey the data using a modulated carrier. The problem with such systems is that the physician must be located near a dedicated receiver that can print a strip chart. If the physician is not located near such a receiver, those treating the patient must describe orally the patient's condition, which again introduces a chance for error. Further, dedicated receivers of this type are generally not standardized and therefore work only with ECG transmitters made by the same manufacturer.

In order to overcome the shortcomings of the prior art patient monitoring devices and to reduce the likelihood of error when transmitting information regarding a patient's condition, the present invention produces a patient report that is transmitted to a remote facsimile machine of the modern "group 3" type. The relatively low cost of facsimile machines, coupled with their high degree of standardization, makes the facsimile format ideal for transmitting patient information monitored by medical instruments.

### Summary of the Invention

The present invention comprises an instrument for recording and transmitting physiological data for a patient. Means are provided for sensing physiological data of a patient and for recording event data indicative of the operation of the instrument. The instrument further includes means for generating a patient report including the physiological and event data in a format suitable for facsimile transmission. Memory means are provided for storing a template of the patient report into which the physiological and event data are inserted. The instrument also preferably includes an ECG monitor for recording the patient's ECG signal and a cardiac defibrillator for providing a cardiac defibrillation pulse to the patient. In the preferred embodiment, the means for generating a patient report is programmed to receive facsimile transmissions and produce a bit mapped image that can be printed on a printer means included in the instrument.

### Brief Description of the Drawings

The foregoing aspects and many of the attendant advantages of this invention will become more readily appreciated when taken in conjunction with the accompanying drawings, wherein:
FIGURE 1 is a block diagram of a medical instrument according to the present invention;
FIGURE 2 is a flow chart that shows how the medical instrument generates a patient report according to the present invention; and
FIGURE 3 is a flow chart showing a method for incorporating the physiological data into the patient report.

### Detailed Description of the e Preferred Embodiment

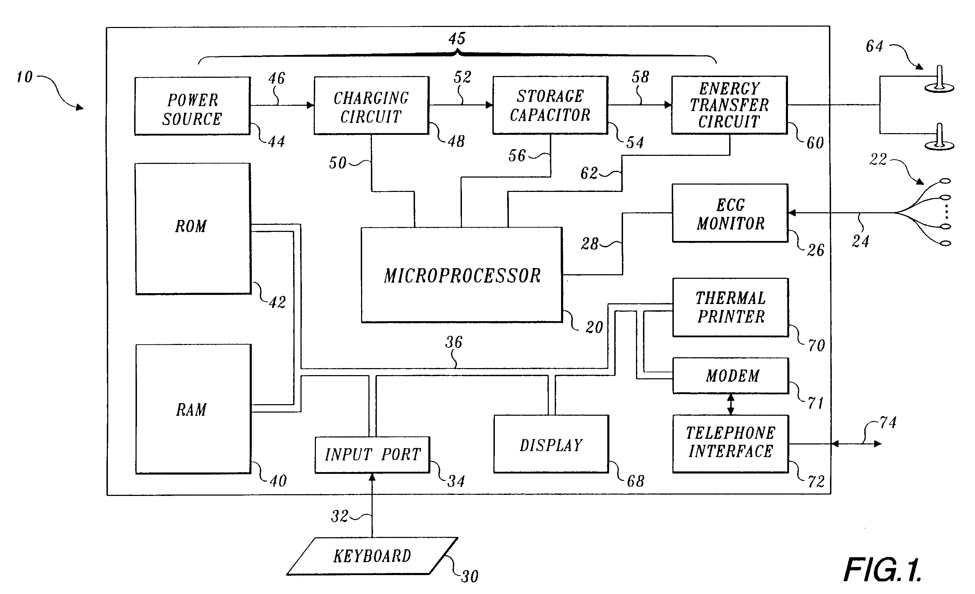

FIGURE 1 is a block diagram of a medical instrument 10 that records physiological data taken from a patient (not shown) and transmits the data to a remote location. A microprocessor 20 is provided within medical instrument 10 to control the operation of the instrument, including recording and transmitting the physiological data. A set of patient electrodes 22 are attached to the patient in order to transmit the patient's physiological signals over a set of leads 24 to an ECG monitor 26. ECG monitor 26 produces digital ECG signals proportional to the patient's electrocardiological signals, which are input to the microprocessor 20 on leads 28. The microprocessor 20 stores the ECG signals in a random access memory (RAM) 40, which is connected to microprocessor 20 by a databus 36. A keyboard 30 is provided so that an operator of the instrument can input information. This information is conveyed from the keyboard over a lead 32 to an input port 34. The input port 34 is connected to microprocessor 20 via databus 36. A read only memory (ROM) 42 is coupled to databus 36 and stores a program that controls the operation of the microprocessor 20. The ROM 42 also stores a template of a patient report that the medical instrument 10 uses when transmitting the physiological data to a remote location in facsimile format as will be described. A display 68 is coupled to databus 36 to provide the operator of the instrument with a visual indication of how the instrument is operating.

In the preferred embodiment, the medical instrument also comprises a cardiac defibrillator shown generally at 45, that is capable of supplying a cardiac defibrillation pulse to the patient in order to terminate ventricular fibrillation. The cardiac defibrillator comprises a power source 44 that supplies energy over a lead 46 to a charging circuit 48. The microprocessor 20 controls the charging circuit 48 with a signal carried via a lead 50, enabling electrical current to flow into a storage capacitor 54 from energy source 44 until the capacitor is sufficiently charged to defibrillate the patient. A lead 56 provides the microprocessor 20 with a signal proportional to the voltage on the storage capacitor 54, and microprocessor 20 stops the charging current flow when a sufficient voltage level has been reached. An energy transfer circuit 60 is controlled by the microprocessor 20 using control signals conveyed on a lead 62 to control the transfer of energy from storage capacitor 54 to a pair of patient electrodes 64 that are applied to the patient. The operation of the cardiac defibrillator, as well as the individual components that comprise it, are well known to those skilled in the medical electronics art and therefore need not be discussed further.

The medical instrument 10 is provided with a telephone interface 72, and a analog modem 71 equipped to specifically handle FAX transmission formats that is connected to the microprocessor 20 on databus 36. The modem 71 is coupled to the telephone interface 72 by a lead 73. The telephone interface 72 couples the medical instrument 10 to a telephone line 74 for transmission of data using methods well known in the electronics communication art. Using the modem 71 and telephone interface 72, the microprocessor 20 can also receive facsimile format data from a remote fax machine printed using a thermal printer 70. The thermal printer 70 is connected to the microprocessor 20 via databus 36.

The microprocessor 20 records both physiological data, such as the ECG signals of the patient, and a series of event codes that indicate the operation modes of the instrument. For example, in the preferred embodiment, the microprocessor 20 is programmed to store in RAM 40 an 8-bit code for each operation the instrument performs. For example, if the set of electrodes 22 that are attached to the patient become disconnected, the microprocessor 20 would store in RAM 40 an 8-bit code identifying that event, as well as storing the time at which the event occurred. Similarly, if the operator of the instrument determined that a cardiac defibrillation pulse was required and set the controls on the instrument to provide a defibrillation, the microprocessor 20 would store in RAM 40 an 8-bit code representing that a defibrillation pulse was required, a time stamp to record the time that such a pulse was delivered, and a code that represents the amount of energy that the defibrillation pulse contained.

The microprocessor 20 is also programmed to produce a patient report that includes any information input by the operator of the instrument via keyboard 30 and the electrocardiological data stored in RAM 40, as well as the event data and time stamps generated as the instrument is operated. In order to produce the patient report, the microprocessor 20 retrieves a template of a patient report from ROM 42 and places the template into RAM 40. The microprocessor then inserts the patient's physiological data, the information entered by the operator, and event data that are stored in RAM 40 to create a bit map of the patient report which is stored in RAM 40. Microprocessor 20 then compresses the bit map of the patient report into a series of codes suitable for transmission to a remote facsimile machine (not shown) via telephone interface 72. In the preferred embodiment, the microprocessor 20 compresses the bit map of the patient report with Group 3 facsimile format standards using conventional Modified Huffman and Modified Read compression algorithms. The compression algorithms used to format the bit map of the patient report into a series of conventional facsimile format codes is well known in the art. For example, such compression schemes are described in Fax: Digital Facsimile Technology and Applications by Kenneth R. McConnell, Dennis Bodson and Richard Schaphorst, Artech House, 1989.

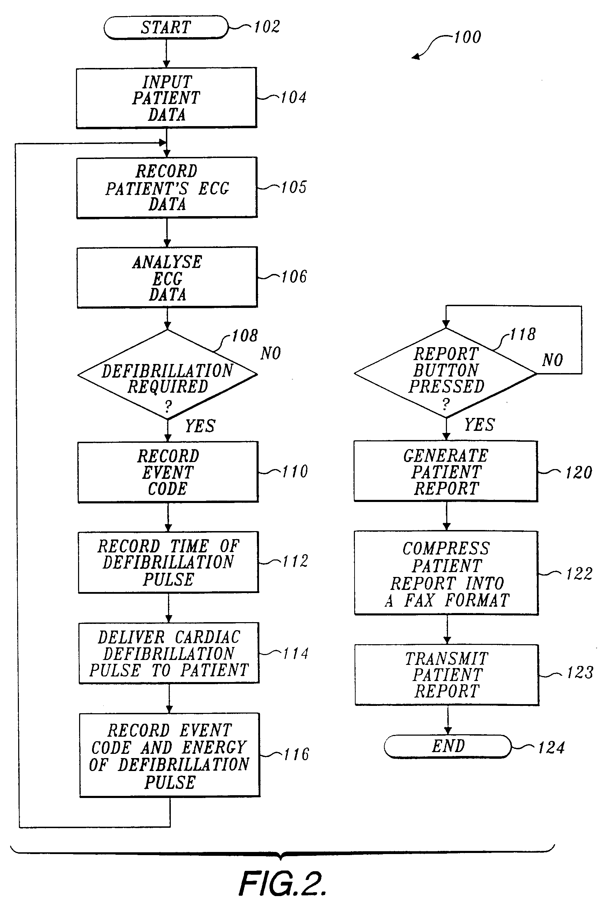

FIGURE 2 is a flow chart of a computer program 100 that operates the medical instrument according to the present invention. The program 100 begins at a start block 102 and proceeds to a block 104 in which the operator of the instrument inputs patient data. Such patient data are preferably input via the keyboard 30 (shown in FIGURE 1). After block 104, the program proceeds to a block 106 wherein the patient's electrocardiographic data are recorded. In the preferred embodiment, a ECG signal is obtained from the patient by sampling the signals from the ECG electrodes 22 at 240 samples/second for 2.7 seconds, producing a total of 648 samples for each of the ECG signals. The sampled ECG signals are stored by the microprocessor 20 in RAM 40 (shown in FIGURE 1).

After block 106, the program proceeds to a decision block 108 in which the medical instrument analyzes the ECG signals, applying criteria well known in the medical electronics art, to determine whether the patient needs defibrillation. If the answer to decision block 108 is "yes," then the program proceeds to a block 110 wherein an event code indicating that defibrillation was required is stored in RAM 40. In a block 112, the program 100 stores a time stamp indicative of the time at which it was determined that the patient needed defibrillation in RAM 40. After block 112, the program proceeds to a block 114 wherein the medical instrument delivers a cardiac defibrillation pulse to the patient. In a block 116, the program 100 stores a event code in RAM 40 indicating that a defibrillation pulse was delivered to the patient, as well as a record of the energy level of the defibrillation pulse delivered. After block 116, the program loops back to a point immediately preceding block 105 in which the patient's ECG signal is again recorded.

Decision block 118 continually polls the keyboard 30 to determine if a patient report should be generated. If a button is pressed that indicates that the operator desires a patient report, a interrupt signal is produced that causes the program to proceed to a block 120.

In block 120, the program 100 generates a pailent report graphically showing the patient's EGG data, as well as a list of the event codes and time stamps. The report may never be displayed to the operator, and may exist only in RAM until finally received and transmitted to a remote facsimile machine. After block 120, the program proceeds to a block 122 wherein the patient report is compressed into a series of facsimile compatible codes suitable for transfer to a remote facsimile machine. This compression can either be performed by the microprocessor 20 or by serially transmitting the completed bit map of the patient data report to a specialized integrated circuit that converts a bit-map display into the proper facsimile codes. Such a integrated circuit is available from Advanced Micro-Devices as a model AM7971A.

After block 122, the program proceeds to a block 124 wherein the patient report is transmitted to a remote facsimile machine (not shown). Such a transmission is made using the telephone interface 72 as shown in FIGURE 1, using methods that are well known to a person of ordinary skill in the art. After block 122, the program ends at a block 124. Although the above description has been directed to a medical instrument that includes a cardiac defibrillator, those skilled in the art will realize that the invention is applicable to other types of medical instruments equally as well and that the patient report could include other types of physiological data, such as heart rate, blood pressure, etc.

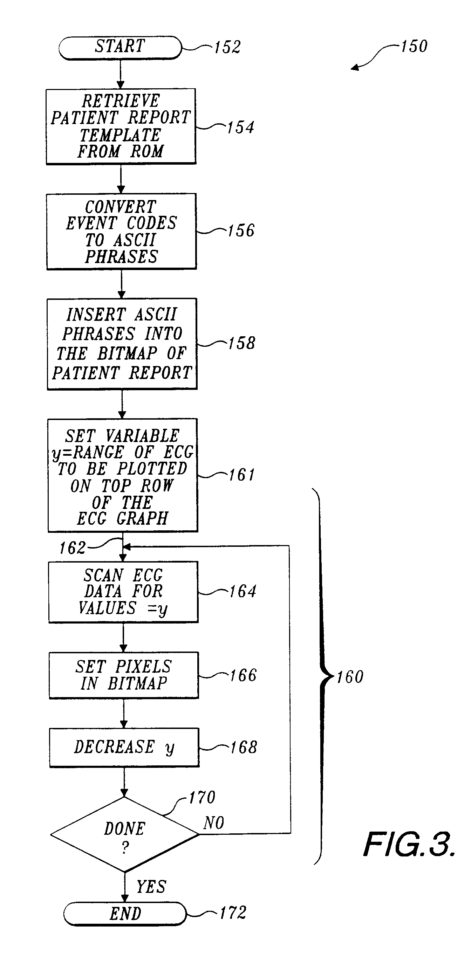

FIGURE 3 is a flow chart of a subroutine 150 that inserts the physiological data, the event data, and time stamps into a bit map of the patient report. The subroutine 150 begins at a start block 152 and proceeds to a block 154. In block 154, the microprocessor 20 retrieves a template of the patient report from the ROM 42. The template of the patient report preferably comprises a bit map having a resolution equal to the resolution of the fax machine to which it is to be transmitted. For example, Group 3 fax machines have 1728 bits per line horizontal resolution and 98 lines per inch vertical resolution. If the template of the patient report is stored as a bit map display without any compression and occupies a full 8-1/2 x 11 inch page, ROM 42 must store at least 233K bytes of data. The template for the patient report is a standard form for all patients and contains blanks into which the physiological data and event data are inserted to customize the report for a particular patient. In a block 156, the microprocessor reads the event codes and time stamps stored in RAM 40 and converts them to ASCII including phrases, numbers, and letters. In a block 158, the subroutine 150 converts the ASCII data into a pattern of pixels using a font lookup table that is stored in ROM 42. In the block 158, the microprocessor 20 inserts the particular patterns of pixels corresponding to the ASCII phrases into the bit map stored in RAM 40. After block 158, subroutine 150 proceeds to a loop 160 that retrieves the stored ECG signal data from RAM 40 and inserts a bitmapped representation of the ECG signal in the patient report.

Loop 160 begins with a block 161 wherein a temporary variable y is set equal to the range of ECG data that can be plotted on the top line of the ECG graph. For example, a typical ECG signal can range from +10 millivolts to -10 millivolts. If the graph of the ECG signal in the patient report is to occupy an area 1½ inches tall and each inch of the patient report includes 98 rows (to be compatible with facsimile standards) then the entire ECG graph must be plotted in an area of 147 rows (1½ inches ·98 rows/inch). Therefore, each row of the graph would represent approximately 0.14 millivolts (20 millivolts ÷ 147 rows). So in this example, y is initially set equal to 9.86(10 - .14) millivolts.

After block 161, the subroutine 150 proceeds to a block 164 wherein the ECG data that are stored in RAM 40 are scanned for values that are greater than or equal to the current value of y. After block 164, subroutine 150 proceeds to a block 166 that turns on an appropriate bit in the bit map of the patient report corresponding to those values of the sampled ECG data that were greater than or equal to the current value of the variable y. For example, if, of the 648 samples of ECG data that comprise the digital ECG signal, samples 200, 400, and 500 have values greater than or equal to the variable y, then bits 200, 400, and 500 in that row of the ECG graph are turned on.

After block 166, subroutine 150 proceeds to a block 168 that decrements the value of the variable y. In this example, y is be decremented to 9.72 (9.86 - .14) millivolts. After block 166, the subroutine 150 proceeds to a decision block 170 that determines if the subroutine is done, i.e., have all the rows of the ECG been graphed? If the answer to decision block 170 is "no," subroutine 150 loops back to a point 162 wherein the stored ECG data are scanned for values that are greater than or equal to the current value of the variable y and have not already been graphed. Loop 160 continues to operate in this manner until each row of the graph has been completed. When the subroutine has finished, the answer to decision block 170 is "yes" and the subroutine proceeds to an end block 172. Those skilled in the computer display art will appreciate that loop 160 operates in much the same way as a raster scan does in computer graphics applications.

As will be apparent to those skilled in the art, the configuration of the patient report template and the information it contains can vary to meet almost any requirement. However, the sampled ECG data and the event codes transmitted by such a system are compatible with many ECG and defibrillator data storage systems currently in use. Thus, the present invention broadly encompasses a method for transmitting physiological data of a patient to a remotely located physician in a format so that the data can be received on a conventional facsimile machine. The transmission need not take place over a telephone line, but can also use a radio-telephone link such as is provided in portable or cellular telephones that are currently available. The wide availability of facsimile machines, coupled with their high level of standardization, makes the above-mentioned method of transmitting physiological data efficient and nearly universally usable.

Although the invention has been described with respect to its preferred embodiment, those skilled in the art will reaize that changes could be made in form and function without parting from the spirit of the invention. Therefore, it is intended that the scope of the invention is to be determined only from the following claims.

## Claims

1. An instrument for recording physiological data indicative of a patient's physical condition and transmitting the physiological data in the form of a patient report to a remote location, comprising:
means for sensing the physiological data;
means for storing the physiological data;
means for generating the patient report that includes the physiological data;
means for formatting the patient report into a set of codes suitable for facsimile transmission; and
means for transmitting the set of codes to the remote location.

2. The instrument as in Claim 1, further comprising:
memory means for storing a template of the patient report, wherein the means for generating the patient report inserts the physiological data into the template of the patient report before it is formatted.

3. The instrument as in Claim 1, wherein the means for storing the physiological data also store event data indicative of a function performed by the instrument such that the means for generating the patient report incorporates the event data into the patient report.

4. The instrument as in Claim 3, wherein the means for storing also store a plurality of time stamps associated with the event data, wherein the time stamps are included in the patient report to indicate the time at which the instrument performed a function.

5. The instrument as in Claim 4, wherein the means for generating the patient report retrieves the template of the patient report and then inserts the physiological data, the event data, and the plurality of time stamps into the template of the patient report.

6. The instrument as in Claim 1, wherein the means for transmitting further comprise a facsimile compatible modem and an interface for coupling the means for transmitting the set of codes to a telephone line that carries the set of codes as they are transmitted to the remote location.

7. The instrument as in Claim 1, further comprising:
means for receiving facsimile signals from the remote location; and
printing means for printing a report corresponding to the facsimile signals received from the remote location.

8. The instrument as in Claim 1, further comprising means for delivering a cardiac defibrillation pulse to the patient.

9. The instrument as in Claim 8, wherein the means for delivering the cardiac defibrillation pulse to the patient comprise:
a energy storage capacitor;
a power source connected to the energy storage capacitor for charging the capacitor with a electrical current;
a energy transfer circuit for controlling the delivery of a electrical current from the energy storage capacitor to a pair of defibrillation electrodes; and
a control circuit for controlling the operation of the power source and the energy transfer circuit.

10. The instrument as in Claim 1, wherein the means for sensing the physiological data comprise:
a plurality of electrodes that are attachable to the patient;
a electrocardiogram monitor coupled to the plurality of electrodes for recording the patient's electrocardiological signals and for generating an electrocardiogram signal as a function of the electrocardiological signals, wherein the electrocardiogram signal is included in the physiological data.

11. An instrument for recording physiological signals of a patient and for transmitting the recorded physiological signals to a remote location, comprising:
a charging circuit for charging an energy storage capacitor with an amount of energy to defibrillate the patient;
a energy transfer circuit for controlling the transfer of energy from the energy storage capacitor to a pair of defibrillation electrodes attachable to the patient;
a control circuit for controlling the operation of the charging circuit and the energy transfer circuit;
means for sensing and recording physiological data indicative of the patient's physical condition and means for recording event data indicative of a function performed by the instrument;
means for producing a patient report that includes the physiological data and the event data;
means for formatting the patient report into a format suitable for facsimile transmission; and
transmission means including a [suitable] facsimile compatible modem for transmitting the formatted patient report to a remote location.

12. The apparatus in Claim 11, wherein the means for formatting the patient report produce a set of codes that recreate the patient report when transmitted to the remote location.

13. The apparatus as in Claim 11, further comprising:
memory means for storing a template of the patient report, wherein the means for producing the patient report inserts the physiological data and event data into the template.

14. The apparatus as in Claim 11, further comprising:
means for receiving facsimile data from the remote location; and
means for printing a report corresponding to the facsimile data received.

15. The apparatus as in Claim 11, wherein the means for sensing physiological data comprise:
a plurality of ECG electrodes, wherein said electrodes produce a plurality of signals corresponding to a patient's electrocardiological signals.

16. The apparatus as in Claim 15, wherein the means for recording the physiological data comprise:
a electrocardiogram monitor coupled to the plurality of ECG electrodes, wherein the electrocardiogram monitor generates ECG signals corresponding to the patient's electrocardiological signals.

17. The apparatus as in Claim 11, wherein the means for recording the event data records a time stamp that indicates the time at which the instrument performed a function.

18. A method for recording physiological data indicative of a patient's physical condition in a medical instrument and transmitting the physiological data to a remote location, comprising:
sensing the physiological data;
storing the physiological data;
formatting the physiological data into a set of codes suitable for facsimile transmission; and
transmitting the set of codes to the remote location.

19. The method of Claim 18, wherein the step of formatting the physiological data further comprises the steps of:
generating a patient report that includes the physiological data; and
transforming the patient report into a set of codes that will reproduce the patient report when transmitted to a remote facsimile machine.

20. The method of Claim 19, wherein the step of generating the patient report further comprises the steps of:
retrieving a template of the patient report; and
inserting the physiological data into the template before the patient report is transformed into the set of codes that are transmitted to the remote facsimile machine.

21. The method of Claim 20, wherein the step of generating a patient report further comprising the steps of:
recording event data indicative of each function performed by the medical instrument;
recording a time stamp that is associated with each of the event data and is indicative of the time at which the medical instrument performed a function; and
inserting the event data and the associated time stamps into the template of the patient report.

22. The method of Claim 21, wherein the step of transforming the patient report further comprises the step of:
compressing the patient report that includes the physiological data, the event data, and the time stamps into a series of Group III facsimile codes, which can be serially transmitted over a telephone line to produce a copy of the patient report when received by a remote facsimile machine.
